(19) Europäisches Patentamt
European Patent Office
Office européen des brevets

(11) Publication number: 0 142 193 A1

# (12) EUROPEAN PATENT APPLICATION

(21) Application number: 84201502.6

(22) Date of filing: 17.10.84

(51) Int. Cl.⁴: A 61 K 39/385
C 07 G 3/00, C 07 K 17/10
G 01 N 33/548

(30) Priority: 22.10.83 NL 8303646

(43) Date of publication of application: 22.05.85 Bulletin 85/21

(84) Designated Contracting States: BE CH DE FR GB IT LI LU NL SE

(71) Applicant: AKZO N.V.
Velperweg 76
NL-6824 BM Arnhem(NL)

(72) Inventor: Visser, Nicolaas
De Sering 21
NL-5831 RW Boxmeer(NL)

(72) Inventor: Greven, Hendrik Marie
Lage Lochemseweg 22
NL-7218 PA Almen(NL)

(74) Representative: Douma, Anno Dominicus et al,
Postbus 20
NL-5340 BH Oss(NL)

## (54) Preparation of immunogens consisting of antigens and/or antigenic determinants bound to glycoside-containing carriers.

(57) The invention concerns a method for the preparation of immunogens comprising reacting at least one type of antigens or antigenic determinants which are for example of polypeptide, oligosaccharide or oligonucleotide nature, with carrier complexes consisting of glycosides having a hydrophilic and a hydrophobic part and hydrophobic compounds provided with one or more groups which show reactivity towards said antigens or antigenic determinants. These immunogens are useful as vaccines, and as immunochemical reagents. Prior to use the immunogens may be stabilized (e.g. by cross-linking of the antigens or antigenic determinants) and/or be protected against digestive degradation (e.g. by micro- or macro-encapsulation).

EP 0 142 193 A1

Croydon Printing Company Ltd.

PREPARATION OF IMMUNOGENS CONSISTING OF ANTIGENS AND/OR ANTIGENIC DETERMINANTS BOUND TO GLYCOSIDE-CONTAINING CARRIERS.

The invention relates to the preparation of immunogens consisting of antigens and/or antigenic determinants bound to a carrier and vaccines which contain these immunogens.

Immunization is employed extensively as a way to protect man and animals against infections by pathogenic organisms such as viruses, bacteria, parasites and the like. Active immunization, which provides long-lasting protection, is preferred. Active immunization is effected by introducing antigens of the pathogenic organism into the individual to be protected. Mostly this is done by inoculation with inactivated or attenuated strains of the pathogen.

Inoculation with inactivated strains has the disadvantage that the immunizing power is often rather low due to the partial denaturation of the antigens which occurs during inactivation. Inoculation with attenuated strains of the pathogens entails the risk of a certain pathogenicity of these strains.

Hence, a safer and more efficient method of active immunization is desired. One possibility is, for example, inoculation with preparations which exclusively contain antigens instead of the complete pathogenic organisms. In this case, the achievement of very high purity, and in particular the complete removal of, for example, viral genetic material, is often difficult to attain. As more information continues to become available on the primary and spatial structure of the antigens, ways have been sought of using antigen fragments which are as small as possible, the so-called antigenic determinants, as immunogens for active immunization. An antigenic determinant is a part of the antigen which can act as a recognition site for a particular type of immunoglobulin directed against the antigen. When split off from the complete antigen, such small fragments are often easier to purify than the antigen itself, and are even small enough to permit their synthetic preparation.

However, a major drawback remains that inoculation with freely dissolved antigens and antigenic determinants often results in much too low a level of immunisation.

Significantly better immunization has been achieved by inoculation with an immunogen wherein antigens or antigenic determinants are bound to a carrier. Specifically, known carriers for this purpose are immunogenic macromolecules such as tetanus toxoid, key-hole limpet haemocyanin (KLH), multi-poly(DL-alanyl)-poly(L-lysine) and copolymers of D-aminoacids. The antigens or antigenic determinants, thus rendered more strongly immunogenic, are however still somewhat less immunogenic than, for example, the antigens in their natural form, such as intact microorganisms. Moreover, the macromolecular carriers employed therein are less suitable for human administration and, for example, KLH is commercially hardly attractive because the material is so expensive.

There have now been found novel immunogens which appear to possess substantially better immunizing power than the immunogens wherein the carriers hitherto known are present. The carriers employed in the immunogens according to the invention are moreover non-pyrogenic and commercially more attractive than the known macromolecular carriers.

The preparation of the immunogens according to the invention is characterized in that antigens or antigenic determinants or derivatives thereof are bonded to reactive groups of hydrophobic compounds which are complexed with at least one type of glycosides having a hydrophilic and a hydrophobic part, whereafter the resulting immunogenic particles are optionally stabilized and/or protected against digestive degradation.

Any glycoside having a hydrophobic part and a hydrophilic part can be used. Preferably, saponins are used, such as those described by Tschesche and Wulf, "Chemie und Biologie der Saponine" in Fortschritte der Chemie organischer Naturstoffe, edited by W. Herz, H. Grisebach and G.W. Kirby, Vol. 30 (1973) and in particular the highly polar saponins, and especially the polar triterpene-saponins such as the polar acidic bis-desmosides, for example saponin-extract from Quillaja bark, araloside A, Chikusetsu-saponin IV, Calendula-glycoside C, Chikusetsu-saponin V, Achyranthes-saponin B, Calendula glycoside A, araloside B, araloside C, Putranjia-saponin III, Bersama saponoside, Putranjia-saponin IV, trichoside A, trichoside B, saponaside A, trichoside C, gypsoside, nutanoside, dianthoside C, saponaside D, aescine from Aesculus hippocastonum or sapoalbin from Gypsophilla struthium, and in particular saponin extract from Quillaja saponaria Molina, preferably the DQ-extract prepared by the method of K. Dalsgaard ("Saponin Adjuvants", Bull.Off.int. Epiz 77 (7-8), 1289-1295 (1972)) and Quil A prepared by the method of K. Dalsgaard ("Saponin Adjuvants III", Archiv für die gesamte Virusforschung 44, 243-254 (1974)).

Mixtures of glycosides may also be used.

According to the present invention antigens or antigenic determinants are used in the preparation of immunogens.

As antigens can be used macromolecular compounds like proteins or polypeptides, polysaccharides or oligosaccharides, glycoproteins, lipoproteins and polynucleotides. These antigens

may be naturally occurring as such, or may be obtained from organisms, in particular from viruses, bacteria, eukaryotic microorganisms, parasites or cells or cell lines belonging to or derived from multicellular organisms. Optionally these antigens may be cleaved enzymatically or chemically prior to use, e.g. in order to remove possibly interfering groups, or to raise the antigenicity. In particular the antigens may also be obtained from recombinant organisms; more particularly recombinant DNA encoded polypeptides are very suitable antigens.

Antigenic determinants suitable for preparing immunogens according to the invented process are for example small polypeptides, small oligosaccharides, oligonucleotides, glycoprotein fragments and haptens.

With small polypeptides are meant here polypeptides comprising at least 4 up to about 40 aminoacids. In general an antigenic determinant comprises not more than 4-8 amino acids. Sometimes, however, a somewhat larger number of amino acids is needed in order to ensure the specific spatial structure of the antigenic determinant. The polypeptides may for example be prepared synthetically on the basis of a known amino acid sequence, or may be obtained by proteolytic or chemical cleavage of larger polypeptides or proteins such as membrane proteins of microorganisms or proteins of plant or animal origin or may be a polypeptide hormone or a fragment or analogue thereof.

With small oligosaccharides in general are meant linear or branched chains of sugar units comprising at least 4 and up to about 20 sugar units, and more favorably between 6 and 10 sugar units. These oligosaccharides can be prepared synthetically or by chemical or enzymic degradation of naturally occurring polysaccharides or glycoproteins.

With oligonucleotides are meant compounds consisting of at least 1 up to about 18 deoxyribonucleotides or ribonucleotides which are obtained synthetically, or by enzymatic or chemical cleavage of polynucleotides such as RNA and DNA. Optionally these oligonucleotides may be doublestranded.

Glycoprotein fragments are prepared synthetically or by chemical or enzymatic cleavage of glycoproteins.

With haptens are meant molecules different from the polypeptides, oligosaccharides, oligonucleotides and glycoprotein fragments mentioned before and which are not suited for the induction of antibody formation unless bonded to a carrier. Examples of these are steroids and prostaglandins.

The antigens or antigenic determinants are to possess at least one reactive group which is suited to directly or indirectly form a bond between said antigen or antigenic determinant and the hydrophobic compounds complexed with the glycosides.

As hydrophobic compound can be used any chemical group with hydrophobic character. Examples of these are hydrophobic polypeptides, or aliphatic compounds or phospholipid derivatives, or cationic detergents.

Hydrophobic polypeptides are suitable hydrophobic compounds in the immunogen according to the invention. These polypeptides can preferably comprise 4-50 amino acids and can in particularly consist of hydrophobic amino acid residues, such as valyl, phenylalanyl, tryptophyl, methionyl, leucyl and/or isoleucyl residues. Inter alia in the interests of appropriate solubility, for example in the course of the synthesis, it may be necessary to include a number of additional hydrophilic amino acids in the hydrophobic compound.

Suitable hydrophobic compounds further are aliphatic straight or branched chain hydrocarbon derivatives, such as alkylamines having about 5-18 carbon atoms, or alkylmercaptans having about 4-15 carbon atoms.

Suitable also are cationic detergents, such as DTAB, DTAC or OTAC, containing additional reactive groups like sulfhydryl, hydroxyl or carboxyl groups.

Advantageously also phospholipids, such as phosphatidyl derivatives containing straight or branched hydrocarbon chains may be used as hydrophobic compounds.

Whichever of the above or other hydrophobic compounds are selected, a general prerequisite is the presence of at least one reactive group such as for example an amino, carboxyl, sulfhydryl, hydroxyl, aldehyde or ketone group. These groups either may form part of the hydrophobic compounds or may be linked thereto by means of a linker following formation of the hydrophobic complex. The nature of the reactive group actually selected primarily depends on the nature of the reactive

groups on the antigen or antigenic determinant to be reacted therewith.

The complexes of the hydrophobic compound and the glycosides with hydrophilic and hydrophobic parts (carrier complexes) can be prepared in numerous possible ways. Stated in most general terms, a solution of the hydrophobic compounds to which, if desired, a solubilizing agent is added, is brought into contact with a glycoside solution which contains at least one glycoside which is composed of one hydrophobic and one hydrophilic part, in an amount at least equal to the critical micelle concentration, and at the same time or subsequently any solubilizing agent present is removed.

Such a preparation can be carried out by dialysing or subjecting to ultrafiltration a solution of the hydrophobic compound, together with a solubilizing agent in the presence of the said glycoside solution.

Specifically for the large-scale preparation of the carrier complex according to the invention, ultrafiltration is a very suitable method.

Advageously the carrier complex may be prepared by mixing both hydrophobic compound and glycosides with hydrophilic and hydrophobic parts in a solvent and applying sonication to this mixture. The carrier complexes subsequently may be separated and/or purified.

However, it is also possible to prepare the carrier complexes by introducing a solution of the hydrophobic compound, in the presence of a solubilizing agent, onto a density gradient of, for example, sugar solutions or salt solutions,

to which such an amount of glycoside containing a hydrophobic part and a hydrophilic part is added that the concentration is at least equal to the critical micelle concentration, and thereafter introducing the whole into a centrifugal force field, whereby the carrier complex is separated out as a band.

The hydrophobic compounds within the carrier complexes thus prepared may or may not be provided with reactive groups suitable for binding of the antigens or antigenic determinants. If reactive groups are present it has to be made sure, by a proper choice of the groups and/or by a proper way of preparing the complexes, that these reactive groups are actually accessable to subsequent coupling of antigens or antigenic determinants.

In particular this can be done using hydrophobic compounds to which the reactive groups are attached via more or less hydrophilic linker groups.

Alternatively the reactive groups may be bonded to the hydrophobic compounds subsequent to the formation of the carrier complexes. Advantageously this can be done by using a bifunctional linker, which on one side attaches to the hydrophobic compound within the complex and on the other side is suited for attachment of the antigen or antigenic determinant.

Suitable bifunctional reagents for cross-linking are described by T.H. Ji (Methods in Enzymology 91, 580-609 (1983)).

As a bifunctional linker advantageously use may be made of a hetero-bifunctional reagent. More in particular are suited bifunctional reagents having one reactive site which reacts with an amino group (such as a succinimidyl group or an imidate group) and another reactive site which reacts with a different group such as a sulfhydryl group (e.g. a maleimido group) or with various groups (e.g. an azidophenyl group, which reacts upon UV radiation) or a dithio pyridyl group.

The invention also includes the preparation of immunogens which comprise various types of antigens and/or antigenic determinants. These can be different antigens and/or antigenic determinants which are characteristic of one and the same antigenic and/or pathogenic entity, if desired for example characteristic for different serotypes of the same pathogen, or different antigens and/or antigenic determinants which are characteristic of different antigenic and/or pathogenic entities.

The immunogens according to the invention can be stabilized, if desired. This can be done, for example, by linking to themselves, or to one another, the antigenic determinants and/or the hydrophobic residual groups bonded thereto ("cross-linking").

The cross-linking can be established by any reagent possessing reactive groups which are able to bond to at least two groups on the antigen or antigenic determinant and/or on the hydrophobic residual group. For the cross-linking of polypeptides generally use is made of glutaraldehyde or formaldehyde. Suitable cross-linking agents

are also homobifunctional reagents (such as bisimidates, bisuccinimidyl esters) or hetero bifunctional reagents (such as compounds which contain an azidophenyl group and an amidate or a succinimidyl or an activated fluorobenzene or a haloketon group).

The immunogen according to the invention is in particular suitable for vaccination. A vaccine containing the said immunogen can inter alia be used for immunization against a particular pathogen, for so-called priming (wherein the body is not immediately stimulated to form specific free antibodies but is effectively preconditioned so that, following infection or vaccination, for example with inactivated or attenuated pathogens, a strong immunisation reaction is brought about; see, inter alia, Emini et al. (1983) Nature 304, 699-703), or for immunological sterilization or castration (whereby antibodies against substances vital to reproductive processes, such as certain hormones, are produced).

The invention therefore also includes vaccines for animal or human use which contain immunogens according to the invention. Such vaccines can be suitable for immunization or for priming against a particular pathogen, with at least one type of antigen or antigenic determinant characteristic of the particular pathogen being present in the immunogen. A plurality of types of antigens and/or antigenic determinants of one and the same pathogen can also be present in the vaccine. At the same time it is possible for each immunogen to comprise a plurality of these antigens and/or antigenic determinants, or for each of the types of antigens and/or antigenic determinants to

be present in separate immunogens. It is also possible, according to the invention, to compose vaccines for a plurality of pathogens, and in that case, once again, the respective antigens and/or antigenic determinants can be present in separate immunogens or conjointly in one immunogen.

The immunogen according to the invention can accordingly also be used in diagnostic tests, for example as a solid phase in a so-called sandwich test, as a particulate phase in an agglutination test, or as a marker phase in an immunochemical test; in the case of the last-mentioned use, the marker is, or can be, included in the immunogen.

To use the immunogen according to the invention for vaccination purposes, the immunogen must be brought into a suitable form for administration. For intramuscular or subcutaneous administration, the immunogens can, for example, be mixed with physiological salt solution. For intranasal or intraocular administration, for example in the form of a spray, an aqueous solution will also preferably be used. For local (for example oral) administration it will in many cases be necessary to use a form which temporarily protects the immunogens (for example against saccharolytic enzymes in the oral cavity or against proteolytic enzymes in the stomach). Such protection may for example be effected by encapsulating the immunogens or by bringing them into a slow-release form or by using one or more aminoacids in the D-form on enzyme-sensitive sites of the polypeptide antigens or antigenic determinants and/or hydrophobic compounds.

By encapsulation is meant any method by which the pharmaceutical is (temporarily) protected against attack by certain desintegrating influences. Encapsulation may be established by covering any individual complex according to the invention with a protecting agent (microcapsules), or by encasing a multitude of complexes into a single protecting shield (macrocapsules).

The encapsulating material may be semipermeable or may become (semi-)permeable if brought within the animal or human body or upon attaining certain sites within the animal or human body. In this way both encapsulation and sustained release may be established by a single pharmaceutical preparation. For the encapsulation, therefore, generally a biodegradable material is used.

For the micro-encapsulation suitably the optionally cross-linked complexes according to the invention are dispersed into a solution of a biodegradable polymer, to which, optionally, surfactants are added. Consequently the polymer is to be build a capsule at the interface. The microcapsules are separated and might be suspended into a physiologically acceptable medium. Alternatively the microcapsules may be lyophylized.

Macro-encapsulation can be established by methods known in the art. Suitably the optionally cross-linked, complexes according to the invention are converted into an appropriate formulation compatible with oral (soft or hard) capsule material, such as gelatine, starch, etc. To this end the complexes to which are added for example mannitol, sucrose, glycerol, etc., may be dried or freeze-dried, whereafter the material can be

granulated or suspended into for example a natural or synthetic gel and filled into capsules which have been enteric coated by, for example, formaldehyde, cellulose acetate phtalate, hydroxypropylmethyl cellulose phtalate or polyacrylic compounds. Alternatively enteric coated capsules are covered internally with a soft hydrophobic layer such as polysiloxane or synthetic or vegetable oils or waxes, prior to filling with the complexes according to the invention in aqueous suspended form.

The invention is further illustrated by reference to the examples below.

## Example 1

IBV protein immunogen

A. Preparation of IBV antigen:

Avian infectious bronchitis virus (IBV) belonging to the group of Corona viruses is purified from the allantoic fluid of IBV-infected 11 day old embryonated eggs by sucrose gradient centrifugation.

The purified IBV is suspended in PBS at 5 mg protein per ml and treated with thermolysin (0.1 mg/ml) in the presence of 5 mM $CaCl_2$ for 15 minutes at 22 $^oC$. The enzymic reaction is stopped by adding 4 mM EDTA (final concentration). By this enzymic reaction the proteineous spikes at the surface of the virus are cut off.

The spikes are separated from the virion particles by centrifugation for 60 minutes at 100.000 xg. The supernatant containing the solubilized spike proteins are diafiltrated in an Amicon ultrafiltration cell equiped with a

PM 30 filter, and subsequently lyophilized.

B. Preparation of saponin Quil A carrier complex:

A concentrated solution of the thiocholine N-n-dodecyl-N,N-dimethyl-N-(2-thioethyl)-ammoniumchloride[$n-C_{12}H_{25}{}^{+}N(CH_3)_2-CH_2-CH_2-SH.Cl^-$], in aqueous ethanol (1:1), is added to a 1% solution of the saponin Quil A in deoxygenated 0.1 M phosphate buffer, pH 6.0, 0.9% in NaCl, 5 mM in EDTA, to a final concentration of 0.4 mM in said thiocholine surfactant. Mixed micelles are formed.

C. Activation of carrier complex:

The mixed micelles prepared according to B. are reacted with 5 mM N-succinimidyl-4-(N-maleimido-methyl)-cyclohexane-1-carboxylate (SMCC) in 0.1 M phosphate buffer pH 5.0, 0.9% in NaCl, 5 mM in EDTA for 30 minutes at room temperature. Excess SMCC is removed by gel filtration on Sephadex G 25 in 0.1 M phosphate buffer, pH 5.0, 0.9% NaCl.

D. Preparation of immunogens:

The activated carried complex obtained under C. is promptly added to a 10 mg/ml solution of the IBV antigen obtained under A., in 0.1 M phosphate buffer, pH 7.4, 0.9% NaCl. The resulting mixture contains Quil A and protein in a weight ratio of 1 to 4. The pH of the solution is adjusted to 7.8 with 0.1 N NaOH, and the mixture is left at room temperature for 2 hours. The immunogens are isolated by centrifuging through a 10-40% by weight of a sucrose gradient at 250.000 g for 10 hours at +4 $^oC$. The complex is then dialysed against 20 mM phosphate buffer pH 7.4, 150 mM NaCl. Particulate immunogen complexes are visible with the electron microscope.

## Example 2

Parvovirus immunogen I

A. Preparation of porcine parvovirus antigen:

The 65 K MW major capsid protein of porcine parvovirus (PPV) is isolated according to the method described by Molitor, T.W., Joo, H.S. and Collett, M.S.: J.Virology 45, 842-854 (1983): PPV containing tissue culture fluid is brought to 25 mM $CaCl_2$ by dropwise addition of concentrated $CaCl_2$ solution. The virus precipitate is spun for 20 minutes at 120,000 xg. The virus containing pellet is subjected to CsCl gradient centrifugation and the virus peak at a density of 1.3 g/ml is pooled, dialysed and treated with 1% sodium dodecyl sulphate (SDS) for 3 minutes. The 65 K protein is isolated by preparative SDS-PAGE, dialysed and freeze-dried.

B. Derivatization of PPV protein:

The protein obtained under A. is dissolved in 0.1 M phosphate buffer, pH 7.5, at 3 mg/ml and treated with 5 molar equivalents of SMCC at room temperature for 30 minutes. Excess SMCC is removed by gel filtration on Sephadex G 25 in 0.1 M phosphate buffer, pH 6.0, 0.9% NaCl, 5 mM EDTA.

C. Preparation of carrier complex:

The $N^{\alpha}$-3-(2-pyridyldithio)propionylated methyl ester derivative of the peptide Lys-Pro-Val-Gly-$(Leu)_5$-Gly in trifluoro-ethanol solution (4%, w/w) is added to a 1% (w/w) solution of the saponin Quil A in 0.1 M acetate buffer, pH 4.5. The resulting mixture, being 2 mM in peptide, is then sonicated to obtain a clear solution. EDTA is added to 5 mM and the solution is flushed with nitrogen. Dithio erythritol is added to a

concentration of 20 mM. The reduction is allowed to take place for 15 minutes at room temperature, upon which the excess of DTE is removed by gel filtration over a Sephadex column equilibrated with a deoxygenated 0.1 M phosphate buffer, pH 6.0, 0.9% in NaCl, also containing 5 mM EDTA.

D. Preparation of immunogens.

The derivatized protein obtained under B. is combined with the thiolated carrier complex obtained under C. in a ratio of 1:4 (Quil A: protein). The solution is left for 5 hours at room temperature, whereon the immunogen complex is isolated by centrifuging in a 10-40% (w/w) sucrose gradient at 250.000 xg for 10 hours at +4 $^{o}$C. The immunogen complexes are subsequently dialysed against 20 mM phosphate buffer pH 7.4, 150 mM NaCl.

## Example 3

### Parvovirus immunogen II

Similar to Example 2 a parvovirus immunogen is prepared starting from the PPV 65 K protein derived by molecular cloning of the respective virus gene in an expression vector in E.coli (Amgen, California, U.S.A.).

## Example 4

### β-endorphin-(6-17) immunogen

1.2 molar equivalents, with respect to the thiocholine compound of a maleimide derivative of β-endorphin-(6-17),

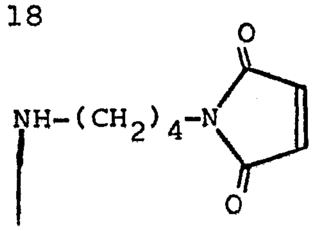

H-Thr-Ser-Glu-Lys-Ser-Glu-Thr-Pro-Leu-Val-Thr-Leu-OH, dissolved in a 0.1 M phosphate buffer, pH 6.0, 0.9% in NaCl, 5 mM in EDTA, are added to the saponin Quil A carrier complex prepared according to Example 1 B.

The mixture is kept at room temperature for 1 hour and is subsequently dialysed against three changes of 20 mM phosphate buffer, pH 7.4, 150 mM in NaCl for 15 hours at 4 °C.

## Example 5

Klebsiella serotype 11 immunogen

A. Klebsiella serotype 11 capsular octasaccharide.

An octasaccharide characteristic of the Klebsiella serotype 11 capsular polysaccharide (K 11 PS) is prepared according to Zigterman et al. (XIIth Internat.Carbohydrate Symp. at Utrecht, the Netherlands; Vonk Publishers, Zeist, 1984; Ed. Vliegenthart et al. p. 308). In summary 5 mg of K 11 PS to which 5 x $10^{10}$ pfu of the bacteriophage Ø 11 in 6 ml of PBS is added is incubated during 24 hours at 37 °C. The octasaccharide (K 11 OS) subsequently is isolated and purified by gel filtration on Sephadex G 25 (Pharmacia). An octasaccharide of the formula I is obtained.

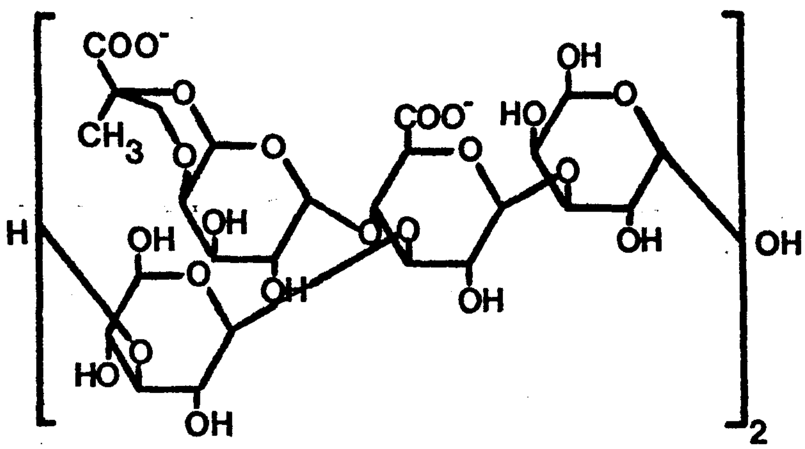

I

B. Activation of K 11 OS

20 mg of the octasaccharide prepared under A. are reacted during 10 days with 25 mg of 2-(4-aminophenyl)-ethylamine while the resulting labile N-alkyl derivative is reduced with sodium cyanoborohydride in 2 ml of aqueous buffer, pH 8.5. After removal of the excess of the reactants by gel permeation chromatography, the product is treated with excess thiophosgene and the resulting isothiocyanotophenyl derivative is freeze-dried.

C. Preparation of carrier complex.

25 mg of phosphatidyl ethanolamine are mixed with 175 mg of the saponin Quil A in a 1% solution in 0.15 M phosphate buffer of pH 7.4 and sonicated during 20 minutes at room temperature. The resulting complexes are extensively dialysed against 0.1 M bicarbonate buffer, pH 9.

D. Preparation of immunogen.

The activated oligosaccharide obtained under B. is dissolved in the carrier complex solution obtained under C. and reacted during 1 week. The product is dialyzed against three subsequent changes of PBS and

freeze-dried.

Example 6

Cross-linked IBV immunogen

To the IBV immunogen prepared according to Example 1 suspended at about 5 mg/ml in 2 ml of 0.1 M phosphate buffer, pH 7.5 is added dropwise, under continuous stirring 1 ml, 20 mM glutaraldehyde. The reaction mixture is left for 30 minutes at room temperature and then chilled in ice. The cross-linked complexes are freeze-dried after extensive dialysis against the above described buffer.

Example 7

Encapsulated canine parvovirus immunogen

Immunogens containing canine parvovirus (CPV) 64 k capsid protein are prepared according to the method described in Example 2.

0.15 g of the above immunogen in 8 g of a 21% albumin solution are mixed with 20 ml of liquid paraffin of 60 $^{o}$C, kept at that temperature during 5 minutes and then cooled to 5 $^{o}$C. This temperature is maintained during 90 minutes. Subsequently 10 g of isopropanol are added. The suspended material is separated by suction filtration on a membrane filter, and washed with isopropanol. Then 3 ml of a 10% formaldehyde in isopropanol solution is added to the particulate material and reacted during 24 hours at 7 $^{o}$C. The hardened encapsulated material is filtered, washed with PBS and stored as a suspension in PBS.

CLAIMS

1. Process for the preparation of immunogens, characterized in that antigens or antigenic determinants or derivatives thereof are bonded to reactive groups of hydrophobic compounds which are complexed with at least one type of glycosides having a hydrophilic and a hydrophobic part, whereafter the resulting immunogenic particles are optionally stabilized and/or protected against digestive degradation.

2. Process according to claim 1, characterized in that two or more different types of antigens and/or antigenic determinants or derivatives thereof are bonded to said reactive group.

3. Process according to claim 1 or 2, characterized in that the immunogenic particles are stabilized by cross-linking of the antigens and/or antigenic determinants or derivatives thereof.
4. Process according to claim 1-3, characterized in that the immunogenic particles are protected against digestive degradation by encapsulation.
5. Vaccine comprising at least one type of immunogen prepared according to one of the processes of the claims 1-4.
6. Immunochemical reagent comprising at least one type of immunogen prepared according to one of the processes of the claims 1-4.

European Patent Office

# EUROPEAN SEARCH REPORT

0142193
Application number

EP 84 20 1502

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 056 249 (NEW YORK BLOOD CENTER, INC.)<br>* Claims 1,14; page 13, line 4 - page 15, line 4; page 15, lines 9-10,15-35 *<br>--- | 1-6 | A 61 K 39/385<br>C 07 G 3/ 00<br>C 07 K 17/ 10<br>G 01 N 33/548 |
| A | GB-A-2 093 039 (NATIONAL RESEARCH DEV. CORP.)<br>* Claims *<br>--- | 1-6 | |
| A | NATURE, vol.290,March 5,1981, pages 51-54, CHESHAM,BUCKS (GB) J. SKELLY et al.:"Hepatitis B polypeptide vaccine preparation in micelle form".<br>* Page 51, right-hand column, lines 3- 21 *<br>--- | 1-6 | TECHNICAL FIELDS SEARCHED (Int. Cl.4)<br>A 61 K<br>C 07 H |
| A | EP-A-0 058 021 (BEECHAM GROUP PLC)<br>* Claims 1,3,4 and 7; page 1, lines 16-29; page 2, lines 19-24; page 4, lines 26-30 *<br>----- | 1-6 | |

The present search report has been drawn up for all claims

| Place of search | Date of completion of the search | Examiner |
|---|---|---|
| THE HAGUE | 06-02-1985 | RIJCKEBOSCH |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82